# EUROPEAN PATENT SPECIFICATION

(11) **EP 2 124 757 B1**
(45) Date of publication and mention of the grant of the patent: **05.10.2016**
(21) Application number: 07712224.0
(22) Date of filing: 15.02.2007
(51) Int. Cl.: A61B 17/02

(54) **IMPROVED ATRIAL RETRACTOR**
VERBESSERTER ATRIUM-RETRAKTOR
RÉTRACTEUR ATRIAL AMÉLIORÉ

(43) Date of publication of application: 02.12.2009
(73) Proprietor: Martens, André Ghislain Annie, 3350 Linter (BE); Epstein, Stephen, Todd, Newtown PA 18940 (US); Sinisi, John Jay, Warminster, PA 18974 (US)
(72) Inventor: MARTENS, André Ghislain Annie, 3350 Linter (BE); EPSTEIN, Stephen, Todd, Newtown, Pennsylvania 18940 (US); SINISI, John Jay, Warminster, Pennsylvania 18974 (US); VANERMEN, Hugo Karel I, 8300 Knokke-heist (BE)
(74) Representative: D'Halleweyn, Nele Veerle Trees Gertrudis
(86) International application number: PCT/EP2007/051466
(87) International publication number: WO 2008/098616

(56) References cited:
- WO-A-96/05773
- US-A- 6 074 343
- US-A1- 2001 004 691
- US-A1- 2002 062 065
- US-A1- 2005 096 508
- US-B1- 6 283 912

## Description

### FIELD OF THE INVENTION

The present invention relates generally to atrial retractors according to the preamble of claim 1 for use in heart surgery, more in particular for minimally invasive heart surgery. Such a device is disclosed in WO-A-96/05773.

### BACKGROUND OF THE INVENTION

The heart consists of a thick muscular layer, the myocardium, which defines four chambers: two upper chambers, the atria, and two lower chambers, the ventricles. The atrium and ventricle on the left side are separated by a mitral valve.

During minimally invasive heart surgery, an atrial retractor is typically used to move portions of the heart tissue and to expose the pulmonary veins and/or the mitral valve. The retraction can be accomplished by using an atrial retractor head that contacts the tissue adjacent to the mitral valve. Placing and setting of an atrial retractor takes a certain amount of time and may damage the heart tissue. It is an object of the present invention to provide an atrial retractor which allows easier placement and less damage of the heart tissue.

US 2005/0228232 discloses a number of improved atrial retractors which are shown in figures 11 and 12 of the specification. While the user of this retractor can immobilize the blade portions relative to each other in a generally fanlike configuration by tightening or loosening a screw, the adjustment of the retractor head is still very limited.

US 2005/0096508 discloses a surgical tool for retracting body tissue during surgery comprising an arrangement with a number of finger-like blades, see e.g. figure 13. The finger-like blades are moveable between a generally compact arrangement with a small radius (to facilitate insertion of the retractor) and a deployed configuration when placed in the heart.
US 6,074,343 discloses a similar retractor with three blades.

US 6,283,912 discloses in figures 33 and 34 a tissue stabilizer.

WO 96/05773 discloses in figure 15 and figure 26 two different embodiments of thorascopic retractors.

The object of the invention is to provide an improved atrial retractor, and an improved retractor head.

### SUMMARY OF THE INVENTION

The present invention provided an atrial retractor which is distinguished by the features of the characterizing portion of claim 1. The atrial retractor is for use in a surgical procedure on a heart, more in particular for minimally invasive heart surgery, comprising an elongated handle, a retractor head and a first hinged connection between the handle and the retractor head. The elongated handle defines an access with first and second ends, the second end being connected to a proximal end part of the retractor head. The hinged connection is so configured that it is adjustable when the retractor head is placed in the heart. The retractor head has a shape and dimensions to be placed in the atrium of the heart to move the heart tissue to create more space at the location to perform surgery on. Such a retractor with hinged connection has the advantage of an improved placement and surgical access during minimally invasive heart surgery when compared to the retractors of the prior art. The hinged connection also allows for more flexibility in external placement of tools and tool positions.

In a further embodiment of the present invention, the first hinged connection is configured in such a way that it is sealed from bio-hazards throughout its full range of motion. This can be achieved by using a hinged connection with two components with contact surfaces that remain in contact during the full range of motion and that seal an internal part of the hinged connection, wherein the adjustment of the angle of the blade relative to the handle is done in said internal cavity. According to an alternative embodiment a flexible tube is used to seal the hinged area from bio-materials.

The present invention may be used with an improved retractor head for use in a surgical procedure on a heart comprising a retractor blade and a flap. The flap is connected to a side of the retractor blade via a hinged connection. This hinged connection is preferably adjustable allowing the flap to be locked in a number of predetermined positions while the retractor head is placed in the heart. According to an embodiment the flap can be folded in against the retractor head, so it can be manoeuvred into small places and then opened up to a number of positions allowing it to retract larger areas and odd-shaped areas for improved surgical access.

### BRIEF DESCRIPTION OF THE DRAWINGS

The foregoing and other features and advantages of the invention will be further elucidated with the following description, including the accompanying drawings, wherein:
Figure 1 is a perspective view of an embodiment of the atrial retractor according to an embodiment of the invention;
Figure 2 is an exploded view of the embodiment of figure 1;
Figure 3A is a detailed view of the retractor head end of figure 2:
Figure 3B is a detailed view of the hand grip end of figure 2;
Figures 4A and 4B are views of the hinged connection in two different positions of the retractor head:
   (A) the retractor head is tilted at 20 degrees downward relative to the handle;
   (B) the retractor head is tilted at 10 degrees upward relative to the handle;
Figure 5 is a detailed view of the hand grip end of the atrial retractor of figure 1, wherein the hand grip has been made transparent for clarity purposes;
Figure 6 is a perspective view of a second embodiment of the retractor head of the invention;
Figure 7 is an exploded view of the embodiment of figure 6;
Figures 8A and 8B show a third embodiment of the retractor head of the invention;
Figures 9A and 9B show a fourth and fifth embodiment of the invention; and
Figure 10 shows a sixth embodiment of the invention.

### DESCRIPTION OF THE PREFERRED EMBODIMENT

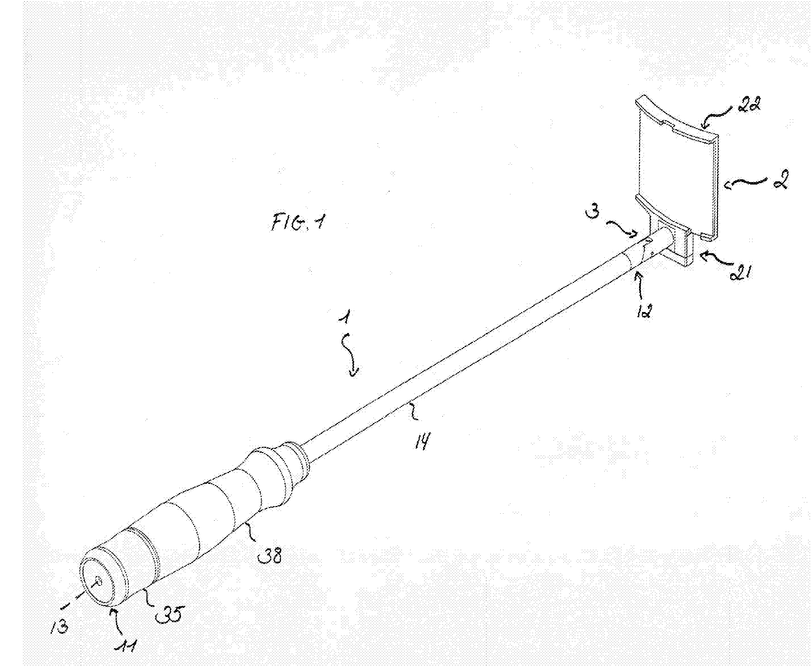

Figure 1 shows a first embodiment of an atrial retractor for use in a surgical procedure on the heart. The atrial retractor mainly comprises an elongated handle 1 and a retractor head comprising a blade 2 with a form adapted to be placed in the atrium, so that the heart tissue can be moved to create more space for a better surgical access to the mitral valve during minimally invasive heart surgery.

The elongated handle 1 defines an axis 13 and has a first end 11 and a second end 12. The retractor head has a proximal end part 21 and a distal end part 22. The proximal end part 21 is connected to the second end 12 of the handle 1 by means of a first hinged connection 3 defining a first pivot axis A1 perpendicular on the axis of the handle 1. The hinged connection 3 allows the retractor head 2 to be tilted at a positive or negative angle for improved placement and improved surgical access during the surgical procedure. The hinged connection 3 also allows for more flexibility in external placement of tools and tool positions.

During a typical operation the retractor head is placed inside the heart at the side of the atrium by means of surgical forceps. Then the handle 1 is introduced through a small opening in the chest and is mounted to the blade 2. To facilitate this mounting, the retractor head comprises a coupling element 4 attached to the retractor head 2 by means of a clamping element 23 and two screws 24. The coupling element 4 is provided with an internal screw thread 25 for cooperation with a corresponding screw thread 26 at the second end 12 of the handle 1. In that way the handle can be simply screwed into the coupling element 4 of the retractor head. Note that any other suitable system may be used for connecting the handle 1 to the retractor head 2. Instead of a screw system, a snap-connection could be used, for example. In that way the second end of the handle can be clicked in the retractor head.

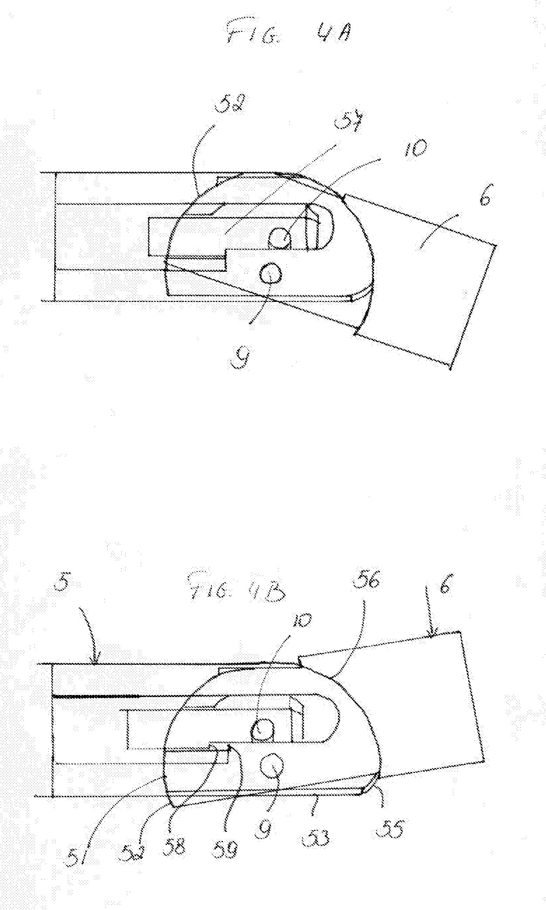

Once the handle 1 is properly attached to the retractor blade 2, the retractor can be manipulated to offer the surgeon a maximum amount of space and visibility. Figures 4A and 4B show two different positions where the retractor blade 2 is tilted at a -20° angle and at a +10° angle relative to the axis 13 of the handle 1, respectively.

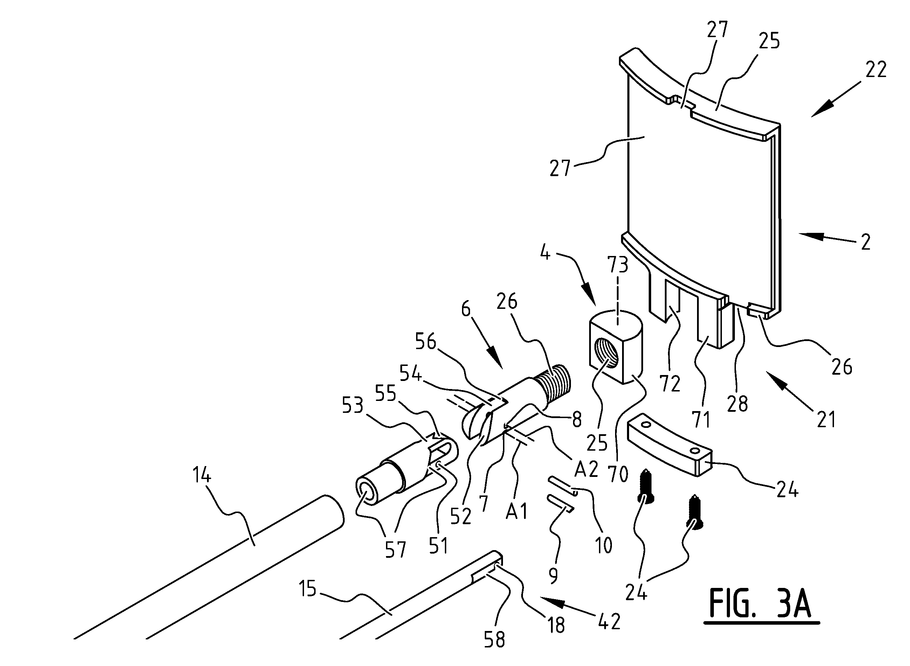

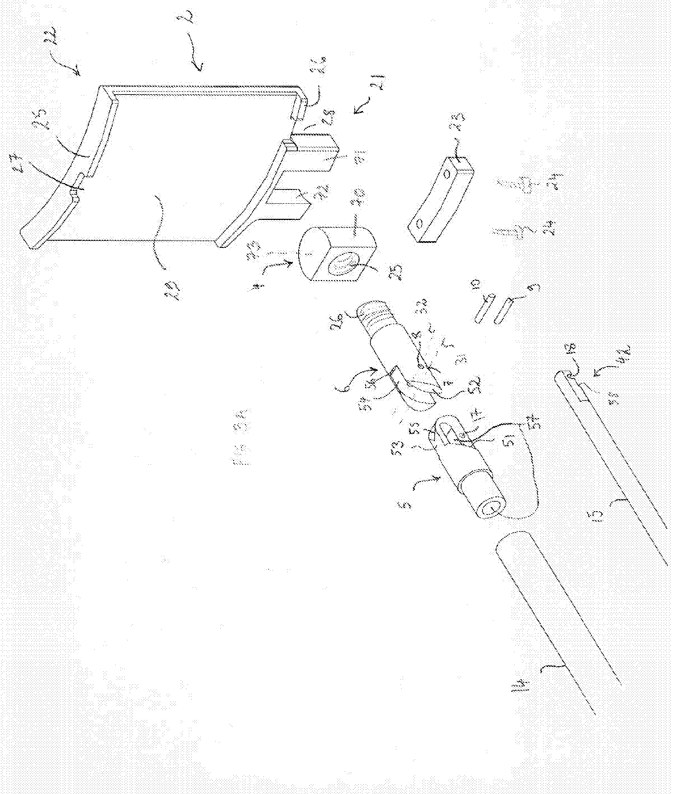

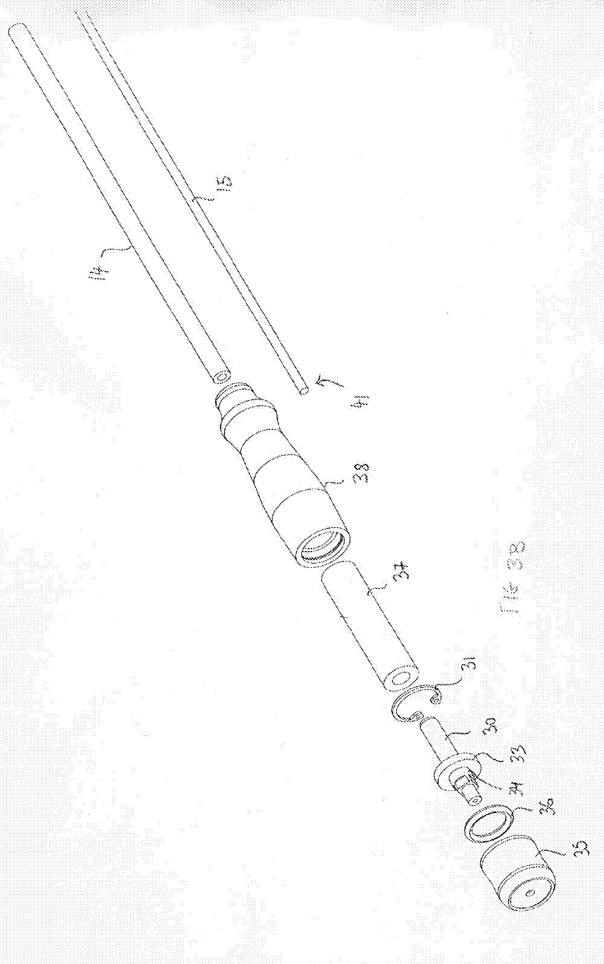

Now the hinged connection 3 and the adjusting means for adjusting the tilting angle will be described in detail referring to figures 3A and 3B. The hinged connection 3 comprises a first coupling component 5 and a second coupling component 6. The skilled person will understand that the first coupling component 5 could be formed as an integral part with the hollow rod 14 of the handle 1. To realize the first hinged connection 3 first pin holes 7, 17 are provided, which extend through the first coupling component 5 and through the second coupling component 6 in a direction perpendicular on the axis 13 of the handle 1. A hinge pin 9 extends through the first pin holes 7, 17.

The first coupling component 5 has a first contact surface 51 and an extension 53 protruding out of said contact surface 51 in the direction of the second coupling component 6. This extension 53 has a third contact surface 55. The second coupling component 6 has a second contact surface 52, and a recess 54 in said second contact surface for receiving the extension 53. The recess 54 has a fourth contact surface 56. The pin hole 7 for hinge pin 9 is provide through the extension 53 perpendicular on the axis 13. In the assembled state the first contact surface 51 abuts against the second contact surface 52, wherein contact between these contact surfaces is maintained during tilting. The same applies for the third and fourth contact surfaces 55, 56. To obtain a maintained contact during tilting, the contact surfaces 51, 52, 55, 56 have a cylindrical shape with a cylinder axis coinciding with the first pivot axis A1. Note that other shapes are possible, such as a spherical shape. It is sufficient if - viewed in a longitudinal section of the components 5 and 6 - the end surfaces describe a circular arc around the pivot axis A1. Such a hinged connection allows for a linear range of motion in both directions (upward motion, figure 4B; downward motion, figure 4C).

Using end surfaces as described above, the hinged connection can be sealed from bio-hazards throughout its full range of motion, as will be further discussed below. Note also that other modifications are possible, such as providing the second coupling component with an extension and the first coupling component with a recess. Also, more than one extension/recess configuration could be envisaged.

The adjusting means for adjusting the angle of the retractor head relative to the axis 13 of the handle 1, comprise an elongated lever 15 with a first end 41 and a second end 42. The lever 15 extends through a hollow section 14 of the handle 1 into a hollow section 57 in the first coupling component 5. The hollow section 57 extends all the way into the extension 53. In that way the second end 42 of the lever 15 can extend into the extension 53 where it can be connected to the second coupling component 6. To connect the lever to the second coupling component, a second hinged connection is used between the lever 15 and the second coupling component 6. Second pin holes 8, 18 are provided which extend through the lever 15 and through the second coupling component 6 in a direction perpendicular on the axis 13 of the handle 1. A second hinge pin 10 extends through the second pin holes 8, 18. This second hinged connection translates an axial movement of the lever 15 into a tilting movement of the blade 2 around the first pivot axis A1 (hinge pin 9). According to a further developed embodiment the remote lever could have additional pivot points for an additional range of motion of the retractor blade 2.

The second end part 42 of the lever 15 is further provided with a cam 58 which abuts against a bearing surface 59 in the hollow section 57, when the blade 2 is positioned in its most downward position, see figure 4A. Note that the location of the cam 58 and the bearing surface 59 is chosen in function of the dimensions of the hollow section 57 in the extension 53 and in function of the dimensions of the contact surfaces 51, 52, 55, 56. The design is such that in the utmost downward position and the utmost upward position of the blade 2, the internal cavity 57 remains sealed. In other words, the dimensions are chosen to ensure that there is never a gap throughout the full range of motion. Note in particular that the underside of contact surface 52 of the second coupling component 6 does not rise above the underside of hollow section 57 - see figure 4A.

The first end 41 of the lever 15 is provided with screw thread for connection to an internally threaded part 30. The internally threaded part 30 is rotatably mounted in hand grip 38 by means of a spacer element 37 and a spring clip 31. The spring clip 31 further ensures that rod 30 is fixed along the axis 13 of the handle 1, and cannot be pushed out of the hand grip 38. The rod 30 is further provided with a bearing surface 33 and a keyed head 34 (here a square head) at the first end 11 of the handle 1. The square head has a screw hole for mounting in the center. The keyed head 34 connects to a knob 35 to allow the device to be more easily manipulated. Further a seal ring 36 is placed between the knob 35 and the bearer surface 33, preventing any bio-hazards from entering the internal threaded rod 30. Turning the knob, the internal threaded rod 30 is rotated adjusting the length of the remote lever 15 both in and out. The screw thread of the rod 30 also works as an automatic lock and stabilizer preventing the lever 15 from moving in or out by itself once the retractor head 2 is correctly positioned.

The skilled person will understand that the internal thread rod mechanism could be replaced with a worn gear mechanism or a paw and racketing device.

The retractor which has been described above referring to figures 1 to 4 further has a self-adjusting retractor head, as will be explained below. The coupling element 4 has a barrel-shaped outer surface 70 and is mounted in the base 71 of the retractor head 2, said base 71 having a recess with a corresponding shape 72. Such a barrel-type hinge 70, 71 allows a limited motion around a vertical axis 73 perpendicular on the axis 13 of the handle 1. The range of motion may vary based on the application. Further a pin or a screw (not shown) may be used to retain the screw thread mount of the second end of the handle 1 in the coupling element 4, while allowing it to pivot. Hence, this barrel-type hinge allows the retractor head 2 to automatically self-adjust its position to more evenly distribute pull weight in relation to the flesh it is retracting. This is advantageous in that it reduces the specific amount of load on any given area by more evenly distributing this load. Reducing the load will also reduce the amount of tissue damage during retraction. This is also a benefit because it allows for more flexibility in the retractor placement and hence a better positioning access to the given surgical area.

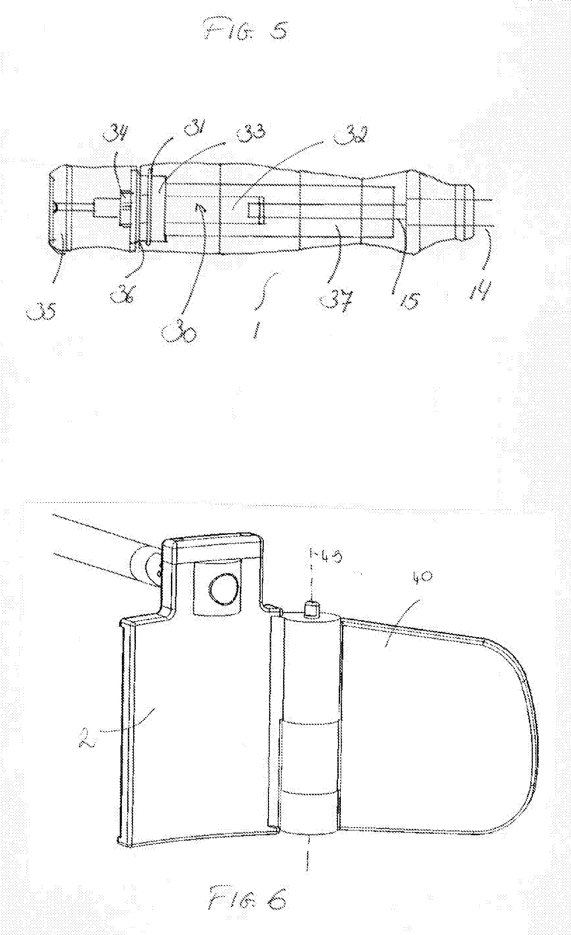

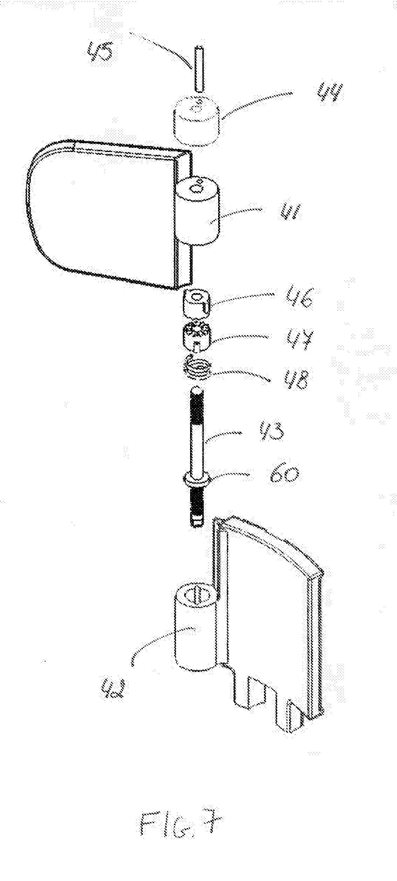

Now a second embodiment of a retractor head will be described in detail referring to figures 6-8. In this second embodiment a pivoting flap 40 is attached to one side of the retractor blade 2. The flap 40 can be folded against the retractor blade 2, so that it can be manoeuvred into small places and then opened up to a number of positions allowing it to retract larger areas and/or odd shaped areas for improved surgical access. Such an improved access will reduce the accidental injury to surgical peripheral areas, will shorten surgical operation time, and will improve overall procedure success rates.

The flap 40 is adjustably connected to one side of the retractor blade 2 using a hinged connection. This is done by means of an adjustable clutch type mechanism 46, 47, 48 allowing the flap 40 to be locked in various predetermined positions. The hinged connection between the flap 40 and the side of the retractor blade 2 uses a first hollow rod 41 at a side of the flap 40 and a second hollow rod 42 at the side of the blade 2, the first rod being in line with the second rod. The second hollow rod 42 is provided with an internal screw thread 61. A pin 43 is screwed inside the second hollow rod and extends through the first rod 41. A cylindrical end element 44 is put on top of the pin 43 extending through the first rod 41. Element 44 and the end of pin 43 are provided with pin holes for receiving a pin 45 for fixing the pin 43 in the first rod 41. The clutch type mechanism comprises a spring 48 and two clutch elements 46, 47 which are fixed against rotation relative to the first and second rod 41, 42, respectively. Pin 43 is further provided with a bearing surface 60, so that the spring 48 can be mounted between the second clutch element 47 and the bearing surface 60. The clutch device is designed in such a way that it is sealed for bio-hazards. This is achieved by integrating the clutch mechanism completely in the two rods 41, 42.

Variations of this hinge type connection may use spring washers, ball detents, and/or disposable plastic parts with keyed snaps and/or locking connections. The main idea is that the flap 40 can be adjusted and locked in different positions relative to the retractor blade 2. The adjustment control of the flap 40 may be activated inside the patient by using forceps to pull open the flap if needed during surgery.

Note further that the retractor blade 2 is provided with edges 25, 26 at its proximal and distal end. These edges may be provided with recesses 27, 28 to facilitate manipulating the retractor blade 2 using forceps. Also the surface 29 of the blade 2 directed towards the handle, may be a rough surface to create more resistance between this surface and the heart tissue for a better grip.

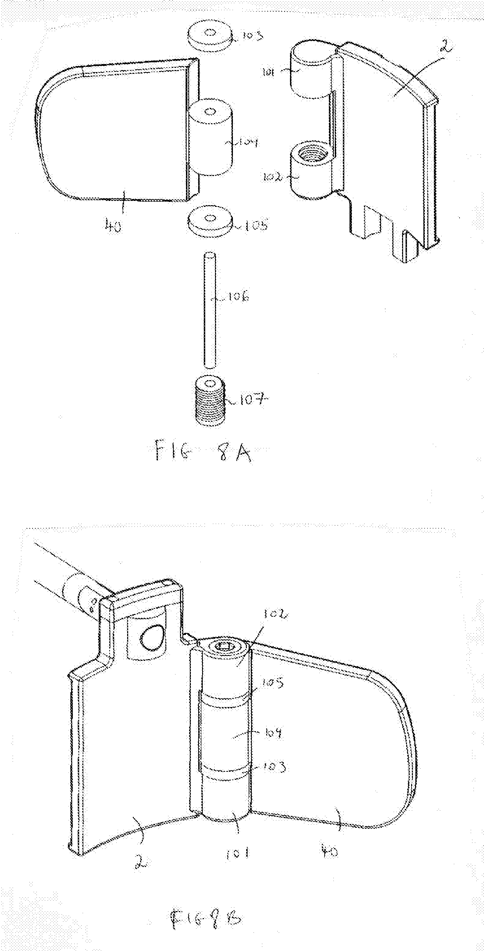

A third embodiment of the retractor head is shown in figures 8A and 8B. In this third embodiment the adjustable flap of friction plate 40 uses a floating pivot pin 106 which is trapped in place by a retaining set screw 107. The plate 2 is provided with two hinge barrels 101, 102, and the flap 40 is provided with a hinge barrel 104 fitting between hinge barrels 101 and 102. Washers 103, 105 are inserted between the hinge barrels. The so formed hinge assembly can be compressed by means of the retaining set screw 107. The tighter this screw is turned, the more friction is generated between the hinge barrels, so that the amount of friction on the hinge can be adjusted for various applications. The washers 103, 105 are used to make the hinge motion more smooth and consistent, but for certain applications the washers could be eliminated. Due to the simplicity of the various parts of this hinge connection, this is a cost effective alternative compared to the second embodiment of the retractor head. Of course, depending on the dimensions and form of the retractor head, it may be preferable to use more than three hinge barrels or to use a different hinge connection.

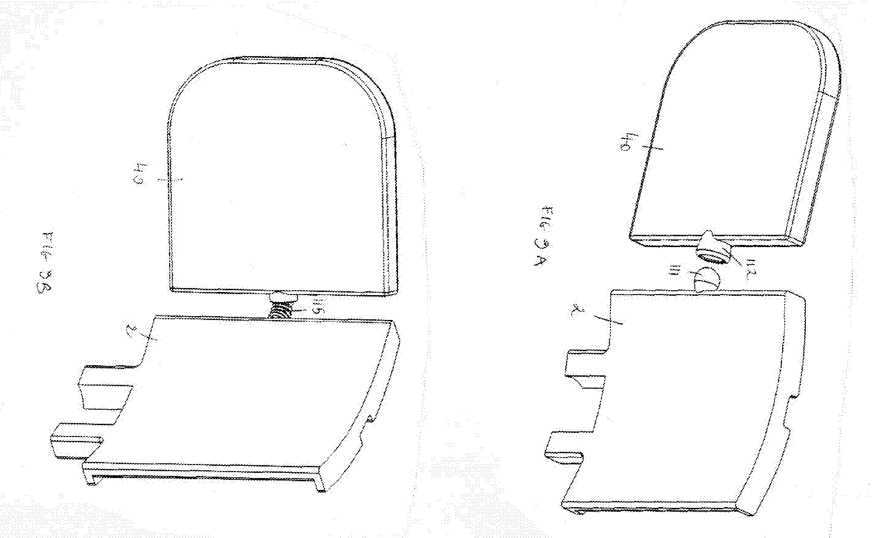

Figures 9A and 9B show a fourth and fifth embodiment of the retractor head of the invention. In the fourth embodiment a ball and socket connection is used. This allows for a three-dimensional movement of the flap 40 relative to the plate 2. As an alternative shown in figure 9B, a bent wire can be used between flap 40 and plate 2. The skilled person will realize that instead of one bent wire multiple flexible wires may be used, and that a spring or spring-like material is also suitable. Such an arrangement allows the flap to be bent and positioned in any direction.

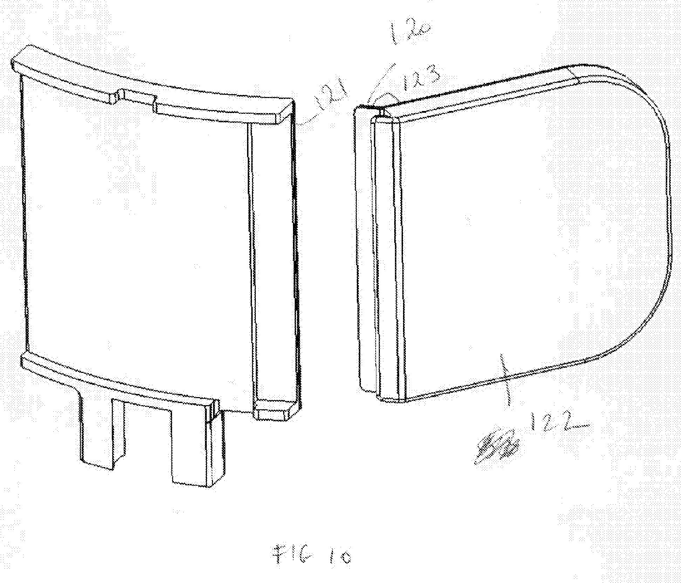

According to yet another embodiment, separate extensions may be attached to the retractor head. For example, instead of the flap 40, separate extensions with various fixed angles could be used, said extensions being configured to be fixed to the retractor head. In that way the same function as that of the hinged flap 40 is achieved, but with less adjustability. Such an embodiment is shown in figure 10. Separate blade extensions 122 with various fixed angles 123 snap to the edge of the retractor blade 2 via an overlapping snap connection 120, 121.

The embodiments described above are examples of devices having elements corresponding to the elements of the invention recited in the claims. Those skilled in the art will understand that it is possible to use embodiments having alternative elements that likewise correspond to the devices claimed. The intended scope of the invention does include other embodiments than the examples given above, and the scope of protection is only determined by the claims.

## Claims

1. Atrial retractor configured for use in a surgical procedure on a heart with a left and a right atrium and a mitral valve, comprising:
an elongated handle (1) defining an axis (13) and having first and second ends (11, 12);
a retractor head (2) comprising a blade having a proximal end part (21) and a distal end part (22), said blade extending in a blade direction between the proximal end part and the distal end part;
said retractor head (2) being configured to be placed in the atrium to move the heart tissue to create more space at the location to perform surgery on;
**characterized in that** the atrial retractor further comprises:
a first hinged connection at the second end (12) of the handle (1), said first hinged connection defining a first pivot axis (A1) perpendicular on the handle (1), and said first hinged connection being configured to be connected to said proximal end of the retractor head such that the first pivot axis is perpendicular on the blade direction, and adjusting means for adjusting the angle of the blade direction of the retractor head (2) relative to the axis (13) of the handle (1) when the retractor head is placed in the heart.

2. Atrial retractor as defined in claim 1, wherein the adjusting means comprise an elongated lever (15), said lever having a first end (41) and a second end (42), said second end (42) of the lever being connected to the proximal end part (21) via a second hinged connection defining a second pivot axis (A2) parallel to the first pivot axis (A1), such that manipulating the lever (15) causes tilting of the retractor head around the first pivot axis (A1).

3. Atrial retractor as defined in claim 2, wherein the handle (1) comprises an elongated hollow section (14), the lever extending through said hollow section.

4. Atrial retractor as defined in claim 3, further comprising an internally threaded part (30) at the first end of the handle (1), said internally threaded part being rotatable around the axis of the handle and fixed against movement in the direction of the axis of the handle, wherein the first end of the lever is provided with screw thread cooperating with the internally threaded part such that a rotation of the internally threaded part causes a movement of the lever along the axis of the handle.

5. Atrial retractor as defined in claim 4, wherein the internally threaded part (30) is provided with a keyed head (34) connecting to a knob (35) at the first end of the handle for rotating the internally threaded rod, and so moving the lever in a longitudinal direction to tilt the retractor head (2).

6. Atrial retractor as defined in any of the previous claims, wherein at least the first hinged connection is sealed against bio-hazards.

7. Atrial retractor as defined in any of the previous claims, wherein the first hinged connection comprises a first part (5) with a first contact surface (51) and an extension (53) protruding out of said end surface, said extension having a third contact surface (55); and
a second part (6) with a second contact surface (52) and a recess (54) therein for the extension of the first part, said recess having a fourth contact surface (56),
the second part pivoting with respect to the first part around the first pivot point,
wherein the first and second end surfaces contact one another and describe a circular course around the first pivot point and
wherein the third and the fourth end surfaces contact one another and describe a circular course around the first pivot point.

8. Atrial retractor as defined in claim 7, wherein first pin holes (7, 17) extend through the extension of the first part and through the second part in a direction perpendicular on the axis of the handle, a hinge pin (9) extending through the first pin holes.

9. Atrial retractor as defined in any of the previous claims, wherein at least the first hinged connection is received in a flexible tube for sealing the hinged connection against bio-hazards.

10. Atrial retractor as defined in claim 2, wherein the first and second hinged connection use a first part (5) with a first end surface (51) and an extension (53) protruding out of said end surface; and a second part (6) with a second end surface (52) and a recess (54) therein for the extension of the first part,
the second part (6) being hingedly connected to the first part (5) around the first pivot axis, wherein the first part has a hollow section (57) for the second end of the lever (15), the second end of the lever extending in said hollow section and being hingedly connected to the second part around the second pivot axis.

11. Atrial retractor as defined in claim 10, wherein the second hinged connection uses second pin holes (8, 18) extending through the lever (15) and through the second part (6) respectively, and a second hinge pin (10) extending through the second pin holes.

12. Atrial retractor as defined in any one of the previous claims, wherein the retractor blade (2) has a number of sides; said retractor further comprising:
a flap (40) being connected to a side of the retractor blade via a third hinged connection defining a third pivot axis (49).

13. Atrial retractor as defined in claim 12, wherein the third hinged connection is adjustable allowing the flap to be locked in a number of predetermined positions while the retractor head is placed in the heart.

14. Atrial retractor as defined in claim 12, wherein the adjustable third hinged connection uses a clutch type mechanism.

## Patentansprüche

1. Vorhofretraktor, der gestaltet ist zur Verwendung bei einer chirurgischen Prozedur an einem Herzen mit einem linken und einem rechten Vorhof und einer Mitralklappe, umfassend:
einen länglichen Handgriff (1), der eine Achse (13) festlegt und der erste und zweite Enden (11, 12) aufweist,
einen Retraktorkopf (2), der ein Blatt mit einem proximalen Endteil (21) und einem distalen Endteil (22) umfasst,
wobei das Blatt in einer Blattrichtung zwischen dem proximalen Endteil und dem distalen Endteil verläuft,
wobei der Retraktorkopf (2) gestaltet ist, um im Vorhof zur Bewegung des Herzgewebes platziert zu werden, damit mehr Platz an der Stelle zur Ausführung einer Operation geschaffen wird,
**dadurch gekennzeichnet, dass** der Vorhofretraktor ferner umfasst:
eine erste Gelenkverbindung an dem zweiten Ende (12) des Handgriffs (1), wobei die erste Gelenkverbindung eine erste Schwenkungsachse (A1) rechtwinklig an dem Handgriff (1) festlegt und wobei die erste Gelenkverbindung gestaltet ist, um mit dem proximalen Ende des Retraktorkopfes derart verbunden zu werden, dass die erste Schwenkungsachse rechtwinklig in der Blattrichtung verläuft,
und eine Einstelleinrichtung zum Einstellen des Winkels der Blattrichtung des Retraktorkopfes (2) relativ zu der Achse (13) des Handgriffs (1), wenn der Retraktorkopf im Herzen platziert ist.

2. Vorhofretraktor nach Anspruch 1, wobei die Einstelleinrichtung einen länglichen Hebel (15) umfasst,
wobei der Hebel ein erstes Ende (41) und ein zweites Ende (42) aufweist und wobei das zweite Ende (42) des Hebels mit dem proximalen Endteil (21) durch eine zweite Gelenkverbindung verbunden ist, die eine zweite Schwenkungsachse (A2) parallel zu der ersten Schwenkungsachse (A1) festlegt, derart, dass eine Handhabung des Hebels (15) eine Neigung des Retraktorkopfes um die erste Schwenkungsachse (A1) bewirkt.

3. Vorhofretraktor nach Anspruch 2, wobei der Handgriff (1) einen länglichen Hohlabschnitt (14) aufweist
und wobei der Hebel sich durch den Hohlabschnitt erstreckt.

4. Vorhofretraktor nach Anspruch 3, ferner umfassend einen Innengewindeteil (30) an dem ersten Ende des Handgriffs (1),
wobei der Innengewindeteil um die Achse des Handgriffs drehbar ist und gegenüber einer Bewegung in Richtung der Achse des Handgriffs fixiert ist,
wobei das erste Ende des Hebels mit einem Schraubgewinde versehen ist, welches mit dem Innengewindeteil derart zusammenwirkt, dass eine Drehung des Innengewindeteiles eine Bewegung des Hebels längs der Achse des Handgriffs bewirkt.

5. Vorhofretraktor nach Anspruch 4, wobei der Innengewindeteil (30) mit einem verkeilten Kopf (34) versehen ist, der mit einem Drehknopf (35) an dem ersten Ende des Handgriffs zur Drehung der Innengewindestange verbunden ist und so den Hebel in einer Längsrichtung zur Neigung des Retraktorkopfes (2) bewegt.

6. Vorhofretraktor nach einem der vorhergehenden Ansprüche, wobei zumindest die erste Gelenkverbindung gegenüber Biogefahren abgedichtet ist.

7. Vorhofretraktor nach einem der vorhergehenden Ansprüche, wobei die erste Gelenkverbindung einen ersten Teil (5) aufweist mit einer ersten Kontaktfläche (51) und einer Verlängerung (53), die aus der Endfläche vorsteht, wobei die Verlängerung eine dritte Kontaktfläche (55) aufweist,
und einen zweiten Teil (6) mit einer zweiten Kontaktfläche (52) und einer Ausnehmung (54) darin zur Verlängerung des ersten Teiles umfasst, wobei die Ausnehmung eine vierte Kontaktfläche (56) aufweist,
wobei der zweite Teil sich in Bezug auf den ersten Teil um den ersten Schwenkungspunkt schwenkt,
wobei die ersten und zweiten Endflächen einander berühren und eine kreisförmige Bahn um den ersten Schwenkungspunkt beschreiben
und wobei die dritten und vierten Endflächen einander berühren und eine kreisförmige Bahn um den ersten Schwenkungspunkt beschreiben.

8. Vorhofretraktor nach Anspruch 7, wobei erste Stiftlöcher (7, 17) durch die Verlängerung des ersten Teiles und durch den zweiten Teil in einer Richtung rechtwinklig zur Achse des Handgriffs verlaufen und wobei ein Gelenkstift (9) durch die ersten Stiftlöcher verläuft.

9. Vorhofretraktor nach einem der vorhergehenden Ansprüche, wobei zumindest die erste Gelenkverbindung in einem flexiblen Rohr zur Abdichtung der Gelenkverbindung gegenüber Biogefahren aufgenommen ist.

10. Vorhofretraktor nach Anspruch 2, wobei die erste und zweite Gelenkverbindung einen ersten Teil (5) mit einer ersten Endfläche (51) und einer aus der Endfläche vorstehenden Verlängerung (53) und einen zweiten Teil (6) mit einer zweiten Endfläche (52) und einer Ausnehmung (54) darin zur Verlängerung des ersten Teiles verwenden,
wobei der zweite Teil (6) mit dem ersten Teil (5) um die erste Schwenkungsachse gelenkig verbunden ist,
wobei der erste Teil einen Hohlabschnitt (57) für das zweite Ende des Hebels (15) aufweist und wobei das zweite Ende des Hebels sich in den Hohlabschnitt erstreckt und mit dem zweiten Teil um die zweite Schwenkungsachse gelenkig verbunden ist.

11. Vorhofretraktor nach Anspruch 10, wobei die zweite Gelenkverbindung zweite Stiftlöcher (8, 18), die durch den Hebel (15) bzw. durch den zweiten Teil (6) verlaufen,
und einen zweiten Gelenkstift (10) verwendet, der durch die zweiten Stiftlöcher verläuft.

12. Vorhofretraktor nach einem der vorhergehenden Ansprüche, wobei das Retraktorblatt (2) eine Anzahl von Seiten aufweist und wobei der Retraktor ferner umfasst:
eine Klappe (40), die mit einer Seite des Retraktorblatts durch eine dritte Gelenkverbindung verbunden ist, welche eine dritte Schwenkungsachse (49) festlegt.

13. Vorhofretraktor nach Anspruch 12, wobei die dritte Gelenkverbindung einstellbar ist, um der Klappe zu ermöglichen, in einer Anzahl von bestimmten Positionen arretiert zu werden, während der Retraktorkopf im Herzen platziert ist.

14. Vorhofretraktor nach Anspruch 12, wobei die einstellbare dritte Gelenkverbindung einen Mechanismus vom Kupplungstyp verwendet.

## Revendications

1. Écarteur atrial configuré pour une utilisation dans un procédé chirurgical sur un coeur avec un atrium gauche et un atrium droit et une valvule mitrale, comprenant :
une poignée allongée (1) qui définit un axe (13) et qui présente des première et seconde extrémités (11, 12) ;
une tête d'écarteur (2) qui comprend une lame qui présente une partie extrémité proximale (21) et une partie extrémité distale (22), ladite lame s'étendant dans une direction de lame entre la partie extrémité proximale et la partie extrémité distale ;
ladite tête d'écarteur (2) étant configurée de façon à être placée dans l'atrium de manière à déplacer le tissu cardiaque de façon à créer plus d'espace au niveau de l'emplacement afin de procéder sur celui-ci à une intervention chirurgicale ;
**caractérisé en ce que** l'écarteur atrial comprend en outre :
une première connexion articulée au niveau de la seconde extrémité (12) de la poignée (1), ladite première connexion articulée définissant un premier axe de pivot (A1) perpendiculaire à la poignée (1), et ladite première connexion articulée étant configurée de façon à être connectée à ladite extrémité proximale de la tête d'écarteur de telle sorte que le premier axe de pivot soit perpendiculaire à la direction de lame ; et
des moyens de réglage destinés à régler l'angle de la direction de lame de la tête d'écarteur (2) par rapport à l'axe (13) de la poignée (1) lorsque la tête d'écarteur est placée dans le coeur.

2. Écarteur atrial selon la revendication 1, dans lequel les moyens de réglage comprennent un levier allongé (15), ledit levier présentant une première extrémité (41) et une seconde extrémité (42), ladite seconde extrémité (42) du levier étant connectée à la partie extrémité proximale (21) par l'intermédiaire d'une deuxième connexion articulée qui définit un deuxième axe de pivot (A2) parallèle au premier axe de pivot (A1), de telle sorte que la manipulation du levier (15) provoque l'inclinaison de la tête d'écarteur autour du premier axe de pivot (A1).

3. Écarteur atrial selon la revendication 2, dans lequel la poignée (1) comprend une section creuse allongée (14), le levier s'étendant à travers ladite section creuse.

4. Écarteur atrial selon la revendication 3, comprenant en outre une partie filetée à l'intérieur (30) au niveau de la première extrémité de la poignée (1), ladite partie filetée à l'intérieur pouvant tourner autour de l'axe de la poignée et étant fixe vis-à-vis d'un déplacement dans la direction de l'axe de la poignée, dans lequel la première extrémité du levier est dotée d'un filetage de vis qui coopère avec la partie filetée à l'intérieur de telle sorte qu'une rotation de la partie filetée à l'intérieur provoque un déplacement du levier le long de l'axe de la poignée.

5. Écarteur atrial selon la revendication 4, dans lequel la partie filetée à l'intérieur (30) est dotée d'une tête clavetée (34) connectée à un bouton (35) au niveau de la première extrémité de la poignée, destiné à faire tourner la tige filetée à l'intérieur, et ainsi à déplacer le levier dans une direction longitudinale de façon à incliner la tête d'écarteur (2).

6. Écarteur atrial selon l'une quelconque des revendications précédentes, dans lequel la première connexion articulée au moins est scellée vis-à-vis de dangers biologiques.

7. Écarteur atrial selon l'une quelconque des revendications précédentes, dans lequel la première connexion articulée comprend une première partie (5) avec une première surface de contact (51), et une extension (53) qui fait saillie hors de ladite surface d'extrémité, ladite extension présentant une troisième surface de contact (55) ; et
une seconde partie (6) avec une deuxième surface de contact (52) et un renfoncement (54) situé à l'intérieur pour l'extension de la première partie, ledit renfoncement présentant une quatrième surface de contact (56) ;
la seconde partie pivotant par rapport à la première partie autour du premier point de pivot ;
dans lequel les première et deuxième surfaces d'extrémité entrent en contact l'une avec l'autre et décrivent un trajet circulaire autour du premier point de pivot ; et
dans lequel les troisième et quatrième surfaces d'extrémité entrent en contact l'une avec l'autre et décrivent un trajet circulaire autour du premier point de pivot.

8. Écarteur atrial selon la revendication 7, dans lequel des premiers trous de broche (7, 17) s'étendent à travers l'extension de la première partie et à travers la seconde partie dans une direction perpendiculaire à l'axe de la poignée, une broche de charnière (9) s'étendant à travers les premiers trous de broche.

9. Écarteur atrial selon l'une quelconque des revendications précédentes, dans lequel la première connexion articulée au moins est reçue dans un tube souple de façon à sceller la connexion articulée vis-à-vis de dangers biologiques.

10. Écarteur atrial selon la revendication 2, dans lequel les première et seconde connexions articulées utilisent une première partie (5) avec une première surface d'extrémité (51), et une extension (53) qui fait saillie hors de ladite surface d'extrémité, et une seconde partie (6) avec une deuxième surface d'extrémité (52) et un renfoncement (54) situé à l'intérieur pour l'extension de la première partie ;
la seconde partie (6) étant connectée par charnière à la première partie (5) autour du premier axe de pivot, dans lequel la première partie présente une section creuse (57) destinée à recevoir la seconde extrémité du levier (15), la seconde extrémité du levier s'étendant dans ladite section creuse et étant connectée par charnière à la seconde partie autour du second axe de pivot.

11. Écarteur atrial selon la revendication 10, dans lequel la seconde connexion articulée utilise des seconds trous de broche (8, 18) qui s'étendent à travers le levier (15) et à travers la seconde partie (6) respectivement, et une seconde broche de charnière (10) qui s'étend à travers les seconds trous de broche.

12. Écarteur atrial selon l'une quelconque des revendications précédentes, dans lequel la lame d'écarteur (2) présente un certain nombre de côtés ;
ledit écarteur comprenant en outre :
un volet (40) connecté à un côté de la lame d'écarteur par l'intermédiaire d'une troisième connexion articulée qui définit un troisième axe de pivot (49).

13. Écarteur atrial selon la revendication 12, dans lequel la troisième connexion articulée peut être réglée, ce qui permet de bloquer le volet dans un certain nombre de positions prédéterminées tandis que la tête d'écarteur est placée dans le coeur.

14. Écarteur atrial selon la revendication 12, dans lequel la troisième connexion articulée qui peut être réglée, utilise un mécanisme du type embrayage.
